# EUROPEAN PATENT APPLICATION

(11) **EP 2 014 333 A1**
(43) Date of publication of application: **14.01.2009**
(21) Application number: 07013673.4
(22) Date of filing: 12.07.2007
(51) Int. Cl.: A61P 27/02, A61K 31/045, A61K 31/125, A61K 31/015

(54) **Use of cooling agents for treatment or prevention of lacrimation or eye burning**

(71) Applicant: Hakiman Shargh Research Company, Isfahan (IR)
(72) Inventor: Mirkheshti, Nooshin, 2nd Robat, Isfahan (IR)
(74) Representative: Fuchs

(57) **Abstract**

The present invention relates to the use of one or more cooling agent selected from the groups of terpenes, carboxamides, menthyl esters, menthone glycerol ketals, terpene analogs, furanones, alkyl substituted urea, sulfonamides, phosphine oxides and combinations thereof, for the manufacture of a medicament for treatment or prevention of lacrimation and/or eye burning.

## Description

The present invention provides novel and advantageous uses of compounds known as cooling agents for the manufacture of a medicament for treatment or prevention of lacrimation or eye burning.

Cooling agents in general are a group of chemical materials known for different applications. These materials can be either from a natural or synthetic source. For example, menthol is a compound than can be obtained from peppermint oil and alternatively menthol or menthol oils can be made synthetically.

They have been added to food and cosmetic products such as chewing gum, mints and creams, as well as to cigarettes, in order to provide a sensation of "coolness" or "freshness" during consumption. The sensation of coolness on the skin and mucosal surfaces resulting from the application of menthol is believed to be due to a specific action on sensory nerve endings. Cooling agents have local anaesthetic and counterirritant qualities. Also, they are contained in combination products which are used for relief of muscle aches, sprains, and similar conditions. The cooling agents have also been added to topical pharmaceutical compositions to alleviate the sensation of inflammation and itch associated with bug bites and mild abrasion.

In WO 200205802 A1 and CN 1416887 A cooling agents are used for preventing and treating cold and influenza.

Also, CN 1456268 A and CN 1554411 A claim rhinitis treating.

JP 11171703 A discloses a pest repellent, which contains the cooling agent menthol.

In addition, DE 1959275 A1 uses cooling agents as mouth deodorant. In particular, a paste against halitosis is described, which contains menthol as cooling agent amongst other ingredients.

JP 11180858 A claims to eliminate foreign body sensation, itching etc. during putting contact lenses on eyes by using these materials. The cooling agents are used in multiform preparations (ophthalmic, nasal, oral and dermal) for different applications.

US2005/0137166 discloses ophthalmic compositions comprising very low concentrations of the cooling agents having a low volatility. The mentioned cooling agents only apply for cooling effect. However, the document US2005/0137166 does not relate to ophthalmic lacrimation and burning effects of the cooling agents included in the compositions. However, the treatment or prevention of lacrimation and/or eye burning is not related to cooling effects, since the efficacy of the treatment/prevention remains even after the cooling effect ends.

Tear flow (lacrimation) consists of a basic secretion and a reflex secretion. The basic secretion is necessary for moisturizing the eye mucosa and the reflex secretion is the response of the eye against exogenous stimulants such as chemical agents (e.g. smoke), physical agents (e.g. intensive light) and mechanical agents (e.g. ocular stroke and wind).

The reflex secretion results from stimulation of parasympathetic nerves of lachrymal glands. In most cases, reflex secretion is unpleasant and tormenting, for example at the time of chopping onions. Also, severe crying can occur due to tear gas and ultraviolet light or due to keratitis or conjunctivitis.

Tears can result from smoke, which is a severe problem for example for fire fighters, and can also be caused by wind, allergens and coryza.

Tears due to allergens and coryza can be controlled by several drugs as anticholinergic drugs, antihistamines and even by anti-inflammatory steroids. But they must be ordered by physicians because of their side effects, which might be severe and may cause discomfort. Therefore, for most cases of tears secretion, the consumption of these drugs should be avoided and is not at all recommendable from a side effect risk point of view.

Therefore it exists a need for providing substances that prevent tear secretion and/or eye burning which might be useful for the preparation of medications obtainable without prescription. Such medications can prevent side effect that occur when active pharmaceutical ingredients (API's) which must be described by a physician are used.

Surprisingly it has been found that cooling agents have an alleviative eye-burning and tears preventive effect besides of providing a "cooling feeling" appreciated by the patient. The feeling of "coolness" as well as the "anti-tears effect" can be provided simultaneously.

The present invention relates to the use of a cooling agent for preparation of ophthalmic, nasal, oral and dermal preparations for treatment or prevention of unpleasant tears secretion (induced by chemical, physical or mechanical stimulants). The use of the cooling agents for the manufacture of the medications leads to compositions that are promptly acting, namely treat or prevent lacrimation or eye burning.

The medication according to the present invention may be applied in any suited form, such as for example in liquid form (like for drop application), which liquid might be a solution. Also suited for application are gels, ointments, creams, pasts or sprays. The compositions may as well be in a form suited for inhalation, like, for example, a cigarette or incense or in a form to be applied with the help of an inhaler, e. g. as an inhalant. Another form of application can be a chewing gum or candy like form. Still further, the medication can also be in the form of a tablet, buccal tablet, capsule, granule, sachet, poultice and so on.

In a special embodiment of the present invention, the compounds being useful as cooling agents are selected from the groups of terpenes, carboxamides, menthyl esters, menthone glycerol ketals, terpene analogs, furanones, alkyl substituted ureas, sulfonamides, phosphine oxides and combinations thereof.

Preferred agents useful in the sense of the present invention are I-menthol, d-menthol, dl-menthol, menthone, alpha-pinene, beta-pinene, d-camphor, dl-camphor, d-borneol, dl-borneol, geraniol, cineol, N,2,3-trimethyl-2-isopropylbutamide, cyclohexanecarboxamide , menthone glycerol acetal, menthyl lactate, (-)-menthoxypropane-1,2-diol, (-)-isopulegol, 4-methyl-3-(1-pyrrolidinyl)-2[5H]-furanone, eucalyptus oil, bergamot oil, fennel oil, rose oil, peppermint oil, mentha oil and others.

The compositions of the present invention will contain one or more of the above described compounds in an amount sufficient to provide an anti-tears effect or eye-burning sensation.

The amount of cooling agent required for each composition will vary depending on the particular application form, that is selected, and the type of composition in which the agent is to be utilized (e.g. inhalant, drops or chewing gum), and other factors apparent to those skilled in the art.

About the amount of cooling agent, in general, the higher the concentration of the cooling agent, the longer it's effect. But high amount of cooling agent is restricted to tolerance level of human and this level depends on releasing rate of the cooling agent from the composition and total amount of absorbed cooling agent. For example, tolerance level of menthol in vaselin base in formulation of ophthalmic ointment is approximately 0.1 %, while this level in nasal ointment is more than 1%. Those formulations which release the cooling agent slowly can contain a higher concentration of cooling agent with a longer effect than formulations which release the cooling agent fast. For instance, addition of polyvinyl alcohol or carboxy methyl cellulose to ophthalmic or nasal drop increase the effect period of cooling agent in preventing of lacrimation or eye burning. Besides, the effectiveness of different cooling agents is very different. For example, the potency of 4-methyl-3-(1-pyrrolidinyl)-2[5H]-furanone is 35 times more powerful in the mouth and 500 times more powerful on the skin than menthol.

Usually, the composition contains 0.0001 to 100 wt.%, preferably 0.001 to 30 wt.%, more preferably 0.006 to 10 wt.%, more preferably 0.01 to 5 and most preferably 0.01 to 2 wt.% of the component or the mixture being useful as cooling agent. In the case of a cutaneous poultice, ointment, cream or gel, the composition preferably contains 0.001 to 3 wt.%, more preferably 0.01 to 2 wt.% of the component or the mixture being useful as cooling agent.

A content of 0.01 to 100 wt.% of any component or the mixture being useful as cooling agent is present in the case of an inhalant and incense.

Usually, the composition contains 1-5000 parts per million (PPM), preferably 60 to 2500 parts per million (PPM) and more preferably 100 to 1000 parts per million (PPM) of the component or the mixture being useful as cooling agent. These amounts are especially useful in the case of a nasal or ophthalmic ointment, cream, gel or drop. In the case of a nasal drop, the composition preferably contains 10-5000 parts per million (PPM), more preferably 60 to 2500 parts per million (PPM) of the component or the mixture being useful as cooling agent. With regard to an ophthalmic drop, the composition preferably contains 1-500 PPM, more preferably 60 to 250 ppm of the component or the mixture being useful as cooling agent.

In the case of a paste, candy, tablet, buccal tablet, capsule, granule or sachet, solution, spray or chewing gum, the dose is 0.0001-10.0 mg for once.

A cigarette according to the present invention contains 0.001 to 2.6 wt.-% of any component or the mixture being useful as cooling agent.

The preferred type of vehicle is an inhalant, candy, paste or chewing gum.

The compounds are preferably non-irritating, non-volatile, and stable in aqueous solutions at physiological pH and osmotic conditions.

The pH of the compositions of the present invention is in one embodiment adjusted to 3.5-10.5 with ordinary methods, preferably by a buffer solution, but it is more preferably adjusted to between 5 and 8 and more preferably between 6 and 7. E.g. the pH properly can be adjusted by the use of any buffer solutions such as citrate, phosphate, etc buffers.

The compositions of the present invention may further contain various additives such as buffering agents, isotonizing agents, solubilizers, and preservatives, viscosity-increasing agents, chelating agents, dispersants, lubricants, fillers and pH regulators.

Examples of the preservatives include chlorobutanol, sodium dehydroacetate, benzalkonium chloride, cetyl pyridinium chloride, phenethyl alcohol, parahydroxybenzoic acid esters, and benzethonium chloride.

The buffers include, for example, borate buffers, phosphate buffers, carbonate buffers, and acetate buffers.

The viscosity-increasing agents include, for example, methylcellulose, hydroxyethylcellulose, carboxymethylcellulose, hydroxypropylmethylcellulose, polyvinyl alcohol, carboxymethylcellulose, chondroitin sulphate, and salts thereof.

The solubilizers include, for example, polyoxyethylene hydrogenated castor oil, polyethylene glycol, polysorbate 80, and polyoxyethylene monostearate.

The chelating agents include, for example, sodium edetate and sodium hydrogen sulphite.

The pH regulators include, for example, phosphate buffer, acetate buffer, citrate buffer, sodium hydroxide, potassium hydroxide, sodium carbonate, citric acid, phosphoric acid, acetic acid and/or hydrochloric acid.

The dispersants include, for example, microcrystalline cellulose and sodium carboxymethylcellulose, sodium alginate, tragacanth, methyl cellulose, magnesium aluminium silicate, xanthum gum, sodium carboxymethylcellulose, sodium starch glycolate, croscarmellose sodium and mixtures thereof.

The lubricants include, for example, magnesium stearate, sodium dodecyl sulphate, silicon dioxide, talc, stearic acid and mixtures thereof.

The fillers include, for example, sugars and sugar alcohols, maltodextrin, starch, hydrolyzed starch, crystalline cellulose, calcium monohydrogen phosphate, light anhydrous silicic acid, titanium oxide, magnesium aluminometasilicate and mixtures thereof.

The carriers include, for example, absolute water, buffers, alcohols, and mineral oils, controlled drug delivery system carriers such as liposomes, niosomes and mixtures thereof.

The compositions are not restricted to the above mentioned materials.

The usage and dose of the compositions of the present invention depends on the severity of the symptoms and the like of the user, but usually 5-10 times at each single application for the use of an inhalant, 1-2 droplets for nasal drops and once for other forms.

In order to protect the eye from chemical agents especially from those causing pH changes, while lacrimation is inhibited according to the present invention, it is recommended to use protective compositions such as pH regulators topically.

The present invention will be described below with reference to the Examples, but is not to be construed to be restricted to these Examples.

Menthol, menthone, alfa-pinene and beta-pinene useful according to the present invention can be, for example, obtained from Merck. Generally, the compounds used according to the invention can be commercially obtained.

### Example 1:

| | |
|---|---|
| Ophthalmic Preparation | |
| I-menthol | 10 mg |
| benzalkonium chloride | 2 mg |
| borax | 10 mg |
| boric acid | 1800 mg |
| sterile distilled water | appropriate amount |
| total volume | 100 ml |

The total volume is made to 100 ml by adding sterile distilled water and the pH of the solution is adjusted to 7.0. The solution is then sterile filtered using a 0.2 µm membrane filter.

### Example 2:

| | |
|---|---|
| Ophthalmic Preparation | |
| beta-pinene | 50 mg |
| benzalkonium chloride | 2 mg |
| borax | 10 mg |
| boric acid | 1800 mg |
| sterile distilled water | appropriate amount |
| total volume | 100 ml |

The total volume is made to 100 ml by adding sterile distilled water and the pH of the solution is adjusted to 7.0. The solution is then sterile filtered using a 0.2 µm membrane filter.

### Example 3:

| | |
|---|---|
| Ophthalmic Preparation | |
| camphor | 10 mg |
| benzalkonium chloride | 2 mg |
| borax | 10 mg |
| boric acid | 1800 mg |
| sterile distilled water | appropriate amount |
| total volume | 100 ml |

The total volume is made to 100 ml by adding sterile distilled water and the pH of the solution is adjusted to 7.0. The solution is then sterile filtered using a 0.2 µm membrane filter.

### Example 4:

| | |
|---|---|
| Inhalant | |
| menthol | 62% w/v |
| peppermint oil | 5% v/v |
| camphor | 5% w/v |
| eucalyptus oil | 6% v/v |
| mineral oil | quantum sufficient to 100% v/v |

The inhaler is made by a vapor nasal inhaler containing a cigarrette shape absorbent filter soaked by the solution.

### Example 5:

| | |
|---|---|
| Inhalant | |
| menthol | 75% w/v |
| mineral oil | quantum sufficient to 100% v/v |

The inhaler is made by a vapor nasal inhaler containing a cigarrette shape absorbent filter soaked by the solution.

### Example 6:

| | |
|---|---|
| Inhalant | |
| camphor | 75% w/v |
| mineral oil | quantum sufficient to 100% v/v |

The inhaler is made by a vapor nasal inhaler containing a cigarette shape absorbent filter soaked by the solution.

### Example 7:

| | |
|---|---|
| Inhalant | |
| beta-pinene | 62% w/v |
| mineral oil | quantum sufficient to 100% v/v |

The inhaler is made by a vapor nasal inhaler containing a cigarrette shape absorbent filter soaked by the solution.

### Example 8:

| | |
|---|---|
| Chewing gum | |
| gum base | 25.0% w/v |
| sorbitol | 45.0% w/v |
| xylitol | 15.0% w/v |
| mannitol | 4.0% w/v |
| glycerin | 9.0% v/v |
| menthol | 2.0% w/v |

Gum base is softened in a gum mixer while being careful not to heat it beyond about 221°F. Once the gum base is pliable, the other ingredients mix into it. Menthol is added last to preserve its character. After mixing, the gum cools slightly before cutting to pieces.

### Example 9:

| | |
|---|---|
| Hard candy | |
| sorbitol | 52.0% w/v |
| gum arabic | 2.0% w/v |
| maltodextrin | 20.0% w/v |
| citric Acid | 1.0% w/v |
| menthol | 2.0% w/v |

Sorbitol (70%) is heated to a boil while using a mixer for agitation. Maltodextrin is added and let the mixture boil. Then, is added Gum Arabic and citric acid. Menthol is added last to preserve its character. After mixing, the hot blend poured into a prepared starch mold.

### Test Examples:

Effect of the cooling agents as anti-tear compounds

### Test 1:

All of the compositions of Examples 1-9 were prepared as well as control compositions of all of them without the cooling agents. Five male and five female adults after obtaining informed consent, were selected as subjects. In a double blind study, they used any of the compositions at every day and then grated 100±10 g onion.

Table 1 shows the mean values of the time before beginning of lacrimation due to onion grating.

**Table 1**

| EXAMPLE | 1 | 2 | 3 | 4 | 5 | 6 | 7 | 8 | 9 |
|---|---|---|---|---|---|---|---|---|---|
| CASE | 414 | 246 | 217 | 1200< | 1200< | 784 | 415 | 576 | 406 |
| CONTROL | 39 | 36 | 41 | 25 | 25 | 30 | 29 | 26 | 28 |

### Test 2:

As test 1, but tears secretion was stimulated by a fixed jet of pressurized air in ten men.

Table 2 shows the mean values of the time before beginning of lacrimation due to wind.

**Table 2**

| EXAMPLE | 1 | 2 | 3 | 4 | 5 | 6 | 7 | 8 | 9 |
|---|---|---|---|---|---|---|---|---|---|
| CASE | 264 | 218 | 265 | 309 | 246 | 296 | 279 | 248 | 284 |
| CONTROL | 91 | 84 | 79 | 94 | 79 | 87 | 94 | 81 | 86 |

## Claims

1. Use of one or more cooling agent selected from the groups of terpenes, carboxamides, menthyl esters, menthone glycerol ketals, terpene analogs, furanones, alkyl substituted ureas, sulfonamides, phosphine oxides and combinations thereof, for the manufacture of a medicament for treatment or prevention of lacrimation and/or eye burning.

2. Use according to claim 1 , wherein the cooling agents are selected from the group consisting of I-menthol, d-menthol, dl-menthol, menthone, alpha-pinene, beta-pinene, d-camphor, dl-camphor, d-borneol, dl-borneol, geraniol, cineol, N,2,3-trimethyl-2-isopropylbutamide, cyclohexanecarboxamide, menthone glycerol acetal, menthyl lactate, (-)-menthoxypropane-1,2-diol, (-)-isopulegol, 4-methyl-3-(1-pyrrolidinyl)-2[5H]-furanone, eucalyptus oil, bergamot oil, fennel oil, rose oil, peppermint oil, and mentha oil.

3. Use according to one or more of the preceding claims, wherein the medicament is an ophthalmic ointment or cream, ophthalmic drops, an ophthalmic gel, a poultice, nasal drops, a nasal ointment or cream, a nasal gel, a nasal aerosol, an oral spray, a mouth washing composition, an oral solution or a chewing gum.

4. Use according to one or more of claims 1 and/or 2, wherein the medicament is an inhalant, incense and/or cigarette.

5. Use according to one or more of claims 1 and/or 2, wherein the medicament is an oral drop, a candy, a paste, a tablet, a buccal tablet, a capsule, granules or a sachet.

6. Use according to one or more of the preceding claims, wherein the medicament contains 0.0001 to 100 wt.-% of the cooling agent.

7. Use according to one or more of the preceding claims, wherein the medicament contains 0.001 to 30 wt.-% of the cooling agent.

8. Use according to one or more of the preceding claims, wherein the medicament contains 0.01 to 2 wt.-% of the cooling agent.

9. Use according to one or more of the preceding claims, wherein the pH of medicament is adjusted to 3.5-10.5 , preferably 5 to 8, more preferably 6 to 7 by a buffer solution.

10. Use according to one or more of the preceding claims, wherein the medicament is applied for the prevention of lacrimation and/or eye burning due to chopping onions, tear gas, smoke, wind, ultraviolet light or hypersensitivity to light.
